# EUROPEAN PATENT SPECIFICATION

(11) **EP 4 680 313 B1**
(45) Date of publication and mention of the grant of the patent: **09.12.2026**
(21) Application number: 24711882.1
(22) Date of filing: 14.03.2024
(51) Int. Cl.: A61M 15/00, A61M 11/00, F16K 15/00

(54) **AN INHALER HAVING A MODIFIED UMBRELLA VALVE**
INHALATOR MIT MODIFIZIERTEM SCHIRMVENTIL
INHALATEUR À CLAPET PARAPLUIE MODIFIÉ

(30) Priority: 15.03.2023 US 202363490363 P
(43) Date of publication of application: 21.01.2026
(73) Proprietor: Vectura Delivery Devices Limited, Chippenham, Wiltshire SN14 6FH (GB)
(72) Inventor: NEFF, Jared, 15068 Lower Burrell (US); EICHELKRAUT, Gero, 01731 Kreischa (DE)
(74) Representative: Clarke, Christopher John
(86) International application number: PCT/EP2024/056788
(87) International publication number: WO 2024/189138

(56) References cited:
- EP-A2- 0 959 281
- US-A1- 2010 095 958
- US-A1- 2013 228 174
- US-A1- 2014 020 680
- US-A1- 2014 116 426
- ANONYMOUS: "Umbrella Valve - Professional Check Valve Supplier", 21 June 2023 (2023-06-21), XP093155706, Retrieved from the Internet <URL:https://web.archive.org/web/20230621083759/https://www.rubbervalve.com/umbrella-valve/> [retrieved on 20240425]
- ANONYMOUS: "Umbrella Valves - Minivalve", 18 February 2020 (2020-02-18), XP093155808, Retrieved from the Internet <URL:https://web.archive.org/web/20200218020615/https://www.minivalve.com/newsite/?option=com_minivalve&product=53&view=minivalve&lang=en&Itemid=52> [retrieved on 20240425]
- ANONYMOUS: "Umbrella Valves - Minivalve", 18 February 2020 (2020-02-18), XP093155812, Retrieved from the Internet <URL:https://web.archive.org/web/20200218020244/https://www.minivalve.com/newsite/?option=com_minivalve&product=48&view=minivalve&lang=en&Itemid=52> [retrieved on 20240425]
- ANONYMOUS: "Silicone Umbrella Valve from China, Silicone Umbrella Valve Manufacturer & Supplier - Better Silicone", 7 December 2022 (2022-12-07), XP093155977, Retrieved from the Internet <URL:https://web.archive.org/web/20221207173715/https://www.better-silicone.com/Silicone-Umbrella-Valve-pl80804.html> [retrieved on 20240425]
- ANONYMOUS: "Umbrella Valve from China, Umbrella Valve Manufacturer & Supplier - Better Silicone", 27 October 2021 (2021-10-27), XP093156226, Retrieved from the Internet <URL:https://web.archive.org/web/20211027100050/https://www.silicongasket.com/Umbrella-Valve-pl594164.html> [retrieved on 20240425]

## Description

The disclosure relates to a breath-controlled inhaler for pulmonary inhalation that has a valve.

Inhalers, which enable the patient to inhale an aerosol, are required for numerous medical applications, for example for the inhalational treatment of asthma, cystic fibrosis (CF) and a range of other respiratory diseases. An aerosol is a dispersion of small solid particles or liquid droplets in a continuous gas phase. Typically, medical inhalational treatments require aerosols of fine droplets of a liquid formulation of a bioactive active ingredient or drug that ideally also reach the tiniest branches of the peripheral lungs, such as bronchioles and alveoli.

In order to achieve the desired homogeneous distribution of droplets in the gas phase, the liquid formulation is atomized in the inhaler by a nebulizer, for example ultrasonic nebulizer, nozzle nebulizer or membrane nebulizer.

Components of the nebulizers currently available are, for example, a reservoir for a liquid or for solid particles, an aerosol generator, a mixing chamber, a mouthpiece, and an airway between an air inlet and the mouthpiece. Depending on the design of the inhaler, there may be yet further components, for example for controlling the airflow.

Various inhalers are known from the prior art:
WO 2006/083014; US 11,534,559; US 11,458,264; US 2021/0252236, US 2022/0126036; and WO 2013/132056.

With these breath-controlled inhalers, an airflow in the inhaler is generated by the patient upon inhalation at the mouthpiece. The airflow mixes with the aerosol generated by the aerosol generator, and the mixture of air and aerosol is transported to the mouthpiece via an air channel. With some inhalers, the patient is supposed to remove the mouthpiece of the inhaler from their mouth during exhalation and to reapply it for inhalation. However, patients frequently exhale into the mouthpiece and hence into the airway in the inhaler. As a result, respiratory air can reach the interior of the inhaler, where it can cause damage in the long run through moisture or, in the case of powder inhalers, moisten or swirl the powder.

Therefore, many of the known inhalers have exhalation valves, which prevent air that is exhaled through the mouthpiece from reaching the interior of the inhaler, in particular the reservoir for liquid or powder, but also the control electronics. This enables the users to exhale into the mouthpiece without causing damage. The exhalation valves used are duckbill valves or flapper valves, for example in US 2022/0126036, US 2021/0252236, and US 11,458,264. During inhalation, when an airflow is generated from the air inlet to the mouthpiece, these valves are open, and during exhalation, when an airflow is generated from the mouthpiece toward the interior of the inhaler, the exhalation valves close. Exhalation valves can also be used to divert the gas flow into the surroundings (environment) during exhalation. US 11,534,559 discloses an umbrella valve as a breath-controlled air inlet valve that opens in the event of a negative pressure in the air inlet chamber of the inhaler.

US 2010/095958 discloses a miniaturized pre-filled, single-use, disposable, small volume nebulizer for medicinal use, said nebulizer employing umbrella type check valves.

Valves which are present in the airway influence the airflow during inhalation, since they are an obstacle in the air channel. Therefore, the valve should be designed in such a way that it affects the airflow as little as possible during inhalation and provides optimal blocking of the airway during exhalation.

The disclosure consists of a breath-controlled inhaler comprising a modified umbrella valve that meets this requirement, and in the use of the modified umbrella valve in the air channel of an inhaler.

The breath-controlled inhaler according to the disclosure comprises
- an air inlet,
- a mouthpiece having a mouthpiece opening,
- an air channel between air inlet and mouthpiece opening, wherein the air channel comprises a mixing chamber,
- a reservoir for a liquid or for solid particles,
- an aerosol generator which is connected to the reservoir and converts the liquid or the solid particles from the reservoir into an aerosol and introduces it into the mixing chamber,
- a modified umbrella valve between the air inlet and the mixing chamber.

The modified umbrella valve is a check valve. It comprises a solid shaft having a first end and a second end. At the first end of the shaft is situated a flexible umbrella. At the second end of the shaft is situated a stop element having an enlarged diameter compared to the diameter of the shaft.

During use, the modified umbrella valve is mounted in the central hole of a perforated plate, the perforated plate being situated in the air channel. The diameter of the flexible umbrella substantially corresponds to the diameter of the air channel or also provides radial sealing thereof via a slight oversize. The diameter of the stop element should be greater than the diameter of the central hole of the perforated plate into which it is inserted. This is the way that the stop element holds the second end of the shaft on the upstream side of the perforated plate. At the same time, the stop element should not be so large that it affects the airflow through the air channel, for example it should cover as few perforations of the perforated plate as possible.

If an air flow in the air channel hits the outside of the umbrella of the umbrella valve (positive pressure), the umbrella is pressed onto the perforated plate and closes the perforations of the perforated plate in axial direction. In addition, an additional seal can be formed by a slight oversize of the outer diameter of the umbrella valve in relation to the inner diameter of the air channel. If, by contrast, a negative pressure is generated on the outside of the umbrella, the umbrella is lifted up from the perforated plate and folds in the direction away from the perforated plate. At the same time, the effective diameter of the umbrella decreases, and a portion of the air-channel diameter is released for an airflow through the perforated plate. The first end of the shaft moves away from the perforated plate until further movement is stopped by the stop element, in accordance with the length of the shaft.

The length of the shaft should be chosen such that the folded umbrella is as far away as possible from the perforated plate in order to affect the airflow through the perforated plate as little as possible. On the other hand, an excessively long distance of the folded umbrella from the perforated plate involves the risk of the umbrella tilting when the negative pressure decreases and not correctly returning to the seat on the perforated plate. According to the invention, the length of the shaft therefore lies in the range from 20% to 50%, particularly preferably in the range from 30% to 40%, of the diameter of the umbrella.

In one embodiment in which the diameter of the umbrella lies in the range from 9.5 mm to 10.5 mm, the shaft is at least 2 mm, preferably at least 2.5 mm, particularly preferably at least 3 mm long. In said embodiment, the shaft is at most 5 mm, preferably at most 4 mm long. Particularly preferably, the shaft in said embodiment is in the range from 3 mm to 4 mm long.

The umbrella of the umbrella valve is preferably composed of silicone or a similarly flexible material having sealing properties. The shaft can consist of a hard plastic such as PEEK (polyetheretherketone) or PC (polycarbonate) or of metal. The stop element can be formed integrally with the shaft, composed of the same material as the umbrella, or it can be attached to the second end of the shaft and composed of a different material. The stop element is one which prevents the second end of the shaft from sliding through the hole of a perforated plate. The umbrella can be formed integrally with the shaft, for example by manufacture by multicomponent injection molding with a form-fit connection or material-to-material bonding, or as a separate component which, for example, is attached to the shaft after the shaft has been fitted in the guide of the perforated plate. The number of individual parts of which the modified umbrella valve consists of depends on how it is to be fitted.

In one embodiment, the modified umbrella valve is mounted in the central hole of a perforated plate. The perforated plate is situated in the air channel between the air inlet and the mixing chamber. The perforated plate can, at the same time, also form the function of an air filter for retention of particles or moisture and comprise an appropriate mesh.

The breath-controlled inhaler preferably comprises an air inlet valve between the modified umbrella valve and the air inlet. The air inlet valve is controlled via a pressure sensor which opens the air inlet valve in the event of a certain negative pressure in the mouthpiece.

The aerosol generator is preferably an electromechanical aerosol generator comprising a vibrating membrane (membrane nebulizer) which seals off the reservoir toward the mixing chamber. As a result of vibration, the aerosol is generated, which mixes with the airflow in the air channel.

In one embodiment, the breath-controlled inhaler according to the disclosure is made up of a head part, a base part, and a mouthpiece. The head part comprises the reservoir and the aerosol generator, and the base part comprises the air inlet and a first section of the air channel. Furthermore, the base part can comprise the air inlet valve, electronics for operation of the aerosol generator, and a pressure sensor. The mouthpiece comprises the mouthpiece opening, the second section of the air channel with mixing chamber, and the modified umbrella valve at the start of the 2nd section of the air channel.

The position of the modified umbrella valve at the entrance of the 2nd section of the air channel prevents possible penetration of exhaled air from the mouthpiece opening right into the 1st section of the air channel in the base part and prevents electronics present there from being damaged by moisture. This could otherwise occur through accidental exhalation into the device by the user. Even if gaskets are present, there may be leaks. This is because of the complexity of the sealing interfaces between components and because of the tolerance difference between the components. This prevents a proper alignment and application of force to elastomer gaskets, which is necessary to ensure leak-tightness. The moisture can also originate from the surroundings, for example if the inhaler is being used in a warm room with high air humidity, after it has been transported at cold temperatures. Damage to the electronics in the base part is particularly critical because the base part is usable multiple times and is intended to remain fit for use for a relatively long period, whereas the mouthpiece and/or the head part may be exchanged regularly. In addition, the position of the modified umbrella valve between air inlet and mixing chamber prevents the aerosol-laden airflow on the way to the mouthpiece opening from being affected.

At the mouthpiece, the patient generates the airflow, into which the aerosol generated by the vibrating membrane is admitted. Via the mouthpiece opening, the mixture of aerosol and air is inhaled by the patient. The necessary air is sucked in via the air inlet opening into the air channel in the base part, from where it reaches the air channel of the mouthpiece.

The present invention is defined by the appended claims.

### Figures and examples

Exemplary embodiments of the disclosure are depicted in the figures and will be more particularly elucidated below. In the figures:
- Fig. 1a: shows an umbrella valve from the prior art in a closed position
- Fig. 1b: shows an umbrella valve from the prior art in an open position
- Fig. 2: shows umbrella valves having a short shaft and having a long shaft
- Fig. 3a: shows a modified umbrella valve in the idle state in the first variant of an air channel of an inhaler
- Fig. 3b: shows a modified umbrella valve during inhalation in the first variant of an air channel of an inhaler
- Fig. 3c: shows a modified umbrella valve during exhalation in the first variant of an air channel of an inhaler
- Fig. 4a: shows a modified umbrella valve in the idle state in the second variant of an air channel of an inhaler
- Fig. 4b: shows a modified umbrella valve during inhalation in the second variant of an air channel of an inhaler
- Fig. 4c: shows a modified umbrella valve during exhalation in the second variant of an air channel of an inhaler
- Fig. 5: shows an exploded drawing of an inhaler from the prior art
- Fig. 6: shows a longitudinal section through an inhaler from the prior art
- Fig. 7: shows measurement results relating to moisture in the inhaler from the prior art without an exhalation valve
- Fig. 8: shows measurement results relating to moisture in the inhaler from the prior art with a modified umbrella valve

### Reference signs

10 - Umbrella valve from the prior art
12 - Shaft end, umbrella valve from the prior art
14 - Shaft, umbrella valve from the prior art
15 - Hook, umbrella valve from the prior art
16 - Umbrella, umbrella valve from the prior art
18 - Perforated plate
19 - Perforation
20 - Modified umbrella valve
23 - Stop element
24 - Shaft
26 - Umbrella
28 - Central hole
29 - Perforated plate or filter plate
100 - Base unit
101 - Air inlet opening
102 - Air outlet opening
103 - Groove
104 - Key locking element
110 - Air channel
118 - Air inlet valve
119 - Pressure sensor
200 - Mouthpiece
200a - First segment of the mouthpiece
200b - Second segment of the mouthpiece
201 - Air intake opening
202 - Lateral opening
203 - Mouthpiece opening
206 - Step
207 - Air channel
208 - Mixing chamber
300 - Aerosol head
301 - Aerosol generator
302 - Reservoir
303 - Key locking element
304 - Lid
309 - Mesh

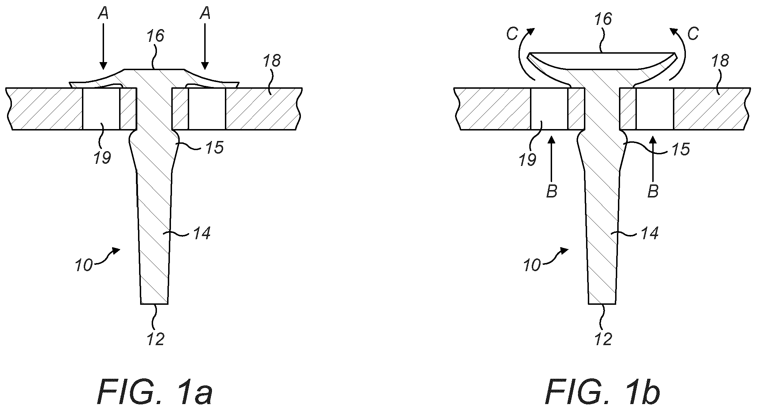

Fig. 1a shows an umbrella valve 10 from the prior art in a closed position. The shaft 14 sits in the central hole of a perforated plate 18, where it is fixed on one of the sides of the perforated plate 18 by the umbrella 16 and on the other side of the perforated plate 18 by the hook 15. The hook 15 is a widening of the shaft 14. The shaft end 12 is spaced from the umbrella 16, so that the shaft 14 of the umbrella valve 10 when fitted can be pulled through the central hole of the perforated plate 18. In the closed position, the umbrella 16 of the umbrella valve 10 closes the perforations 19 in the perforated plate 18. The umbrella valve 10 is in the closed position when a medium (gas or liquid) exerts pressure on the outside of the umbrella 16 (arrows A). From a certain pressure of a medium into the opposite direction through the perforations 19 of the perforated plate 18 onto the bottom side of the umbrella 16 (arrows B), the umbrella 16 folds away from the perforated plate, i.e. the outer regions of the umbrella 16 lift up from the perforations 19, so that the medium (air or liquid) can flow through the perforations 19 (Fig. 1b, arrows C).

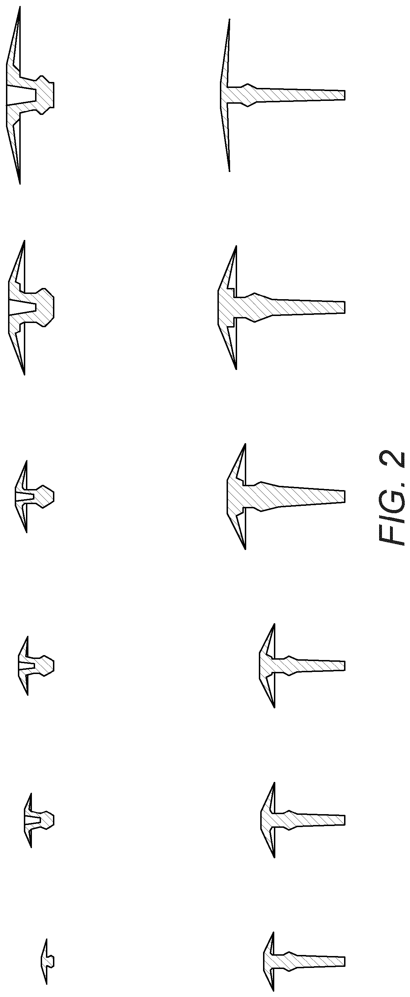

Fig. 2 shows not only umbrella valves having a long shaft, but also umbrella valves having a short shaft. When fitted, they are not pulled through the central hole of a perforated plate, but are one-sidedly pressed in until the hook engages. The shaft serves as a fitting aid and as a support by means of an undercut. It has little axial movability purely for fitting purposes. In the prior art, it does not perform the function of axial slidability under negative/positive pressure in the air channel for better clearance of the flow cross section.

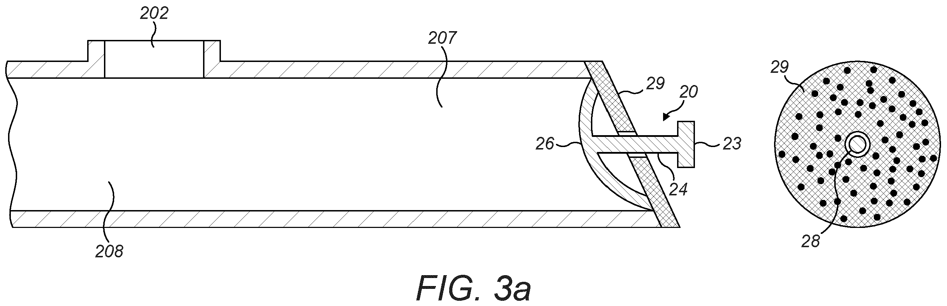

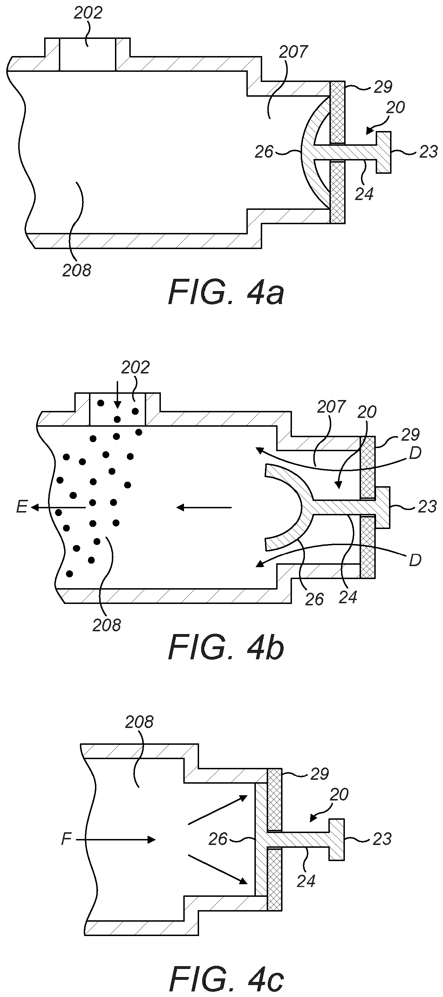

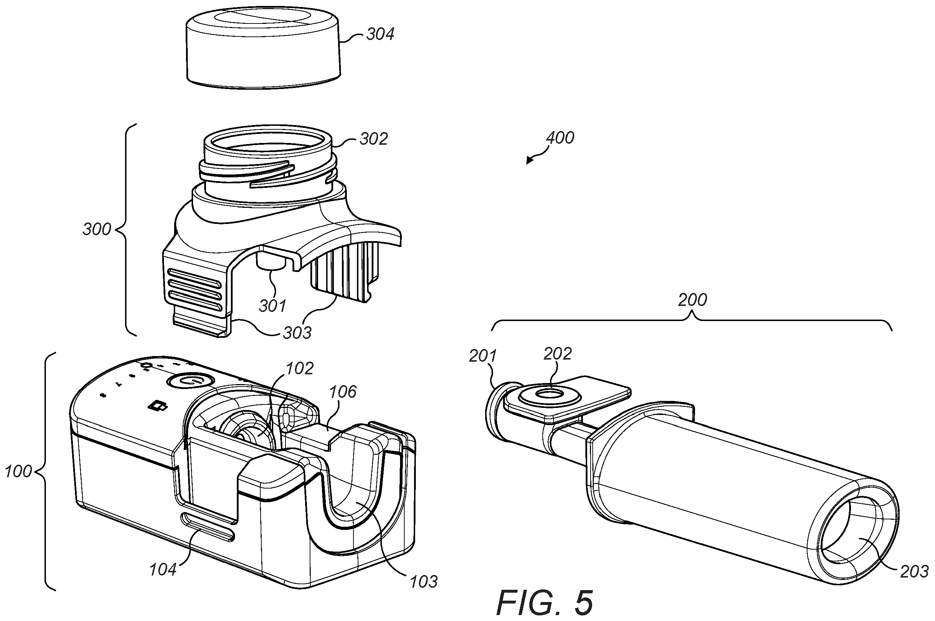

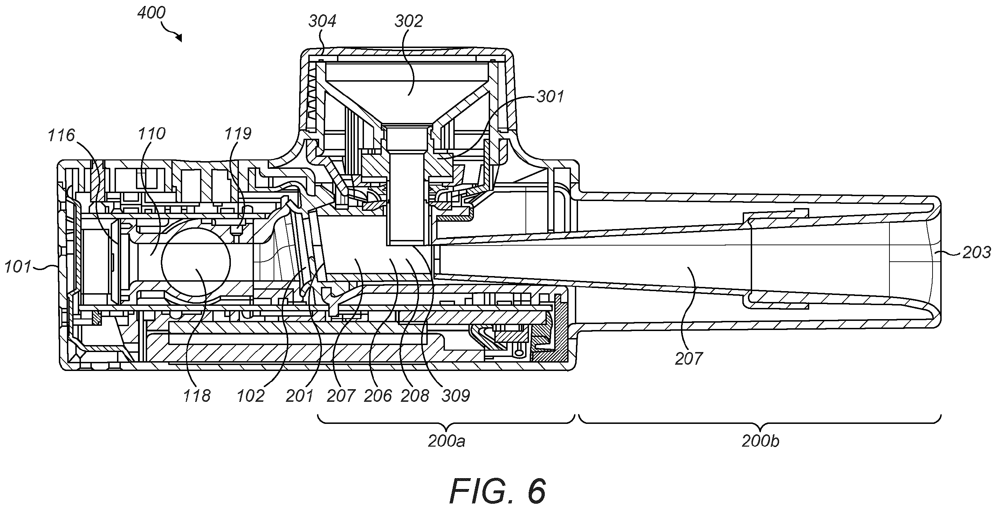

Figs. 3a and 4a show the modified umbrella valve 20 in the idle state in the air channel 207 of an inhaler 400 (Figs. 5 and 6). The two variants of the air channels 207 in Figs. 3a-c and 4a-c differ in that the air channel 207 in Figs. 3a-c has a constant diameter, whereas the air channel in Figs. 4a-c has a step, so that the air channel widens between perforated plate or filter plate 29 and mixing chamber 208.

The modified umbrella valve 20 comprises a shaft 24 and, at the first end thereof, an umbrella 26. The modified umbrella valve 20 differs from a known umbrella valve 10 in that a stop element 23 is situated at the second end of the shaft 24. Furthermore, there is no hook 15 present as in the case of the umbrella valves 10 from the prior art (which holds the shaft in a fixed position relative to the perforated plate or filter plate). In the idle state, the umbrella 26, as a consequence of its natural tension, is arched toward the shaft 24 and is situated in an undefined position in the air channel 207, up to a maximum distance from the perforated plate or filter plate 29 that is limited by the stop element 23. In Fig. 3a and Fig. 4a, the umbrella 26 of the modified umbrella valve in the idle state is situated in a position directly on the perforated plate or filter plate 29. The shaft 24 is mounted in the central hole 28 of a perforated plate or filter plate.

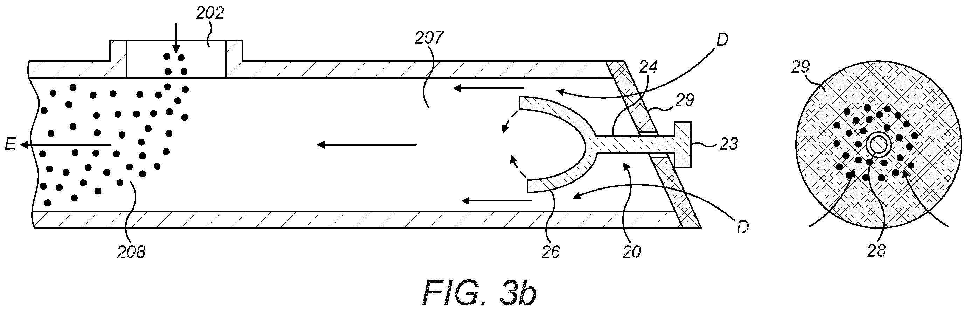

Figs. 3b and 4b show the situation when an airflow is generated in the direction of arrow D through the perforated plate or filter plate 29. The air flows through the perforated plate or filter plate 29 and, in doing so, lifts up the umbrella 26 of the umbrella valve 20. Owing to the air flow, the umbrella 26 is pushed away from the perforated plate or filter plate 29 as far as is allowed by the stop element at the second end of the shaft 24. Thereafter, the umbrella 26 of the umbrella valve 20 folds away from the shaft 24 in the direction of the airflow D. Now, the air flows relatively unimpeded into the mixing chamber 208, in which the airflow mixes with the aerosol from the lateral opening 202 connected to the aerosol generator 301. The air-aerosol mixture then flows in the further direction of the mouthpiece opening 203 of the inhaler (arrow E).

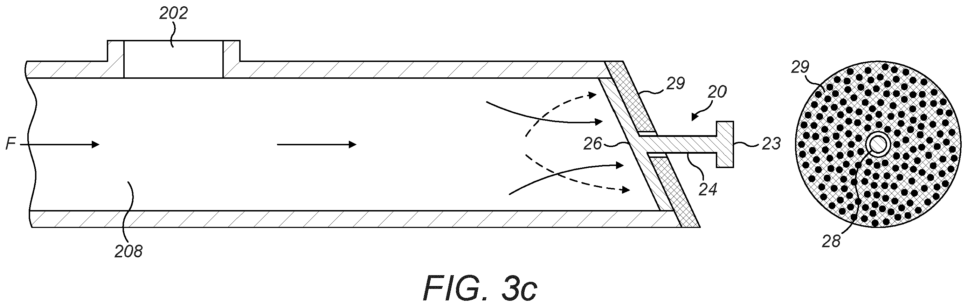

If an airflow is generated in the opposite direction (Fig. 3c/Fig. 4c, arrow F), the umbrella 26 is pressed onto the perforated plate or filter plate 29. At the same time, the umbrella 26 closes the perforations in the perforated plate or filter plate 29 in an axial direction and the airflow comes to a standstill. An additional sealing effect can be produced radially by a slight oversize of the umbrella valve in relation to the inner diameter of the air channel.

Fig. 5 shows an exploded drawing and Fig. 6 shows a longitudinal section of an inhaler 400 from the prior art (EP 2724741) that has been improved by fitting the modified umbrella valve 20 at a suitable position.

The inhaler 400 comprises a base unit 100, a mouthpiece 200 and an aerosol head 300.

The base unit 100 comprises one or more air inlet openings 101, an air outlet opening 102, a groove 103 for accommodation of the mouthpiece 200, and one or more key locking elements 104. The air inlet openings 101 and the air outlet opening 102 are connected inside the base unit 100 to form an air channel 110. Under operating conditions and during the inspiration phase of a user, air flows from the upstream end toward the downstream end of the air channel 110, i.e., from the air inlet openings 101 to the air outlet opening 102.

A pressure sensor 119 measures the negative pressure of inhalation and activates the aerosol generator 301 from a certain minimum pressure and opens/closes the air inlet valve 118 in order to control the airflow. The pressure sensor 119 is in contact with the airway as an orthogonal bypass in relation to the main gas flow of the airflow in the air channel 110.

The mouthpiece 200 comprises a first segment 200a, comprising an air intake opening 201 attachable to the air outlet opening 102 of the base unit 100, and a lateral opening 202 for connection to the aerosol generator 301. Following the air intake opening 201 is an air channel 207, which expands in the region of the lateral opening to a mixing chamber 208. In the mixing chamber 208, during use, the air mixes with the aerosol mixture from the aerosol head 300. The first segment 200a is insertable into the groove 103 of the base unit 100. A second segment 200b is situated downstream of the first segment 200a and comprises the continuation of the air channel 207 from the first segment 200a and the mouthpiece opening 203.

The aerosol head 300 comprises an aerosol generator 301, a reservoir for a liquid 302, and one or more male or female key locking elements 303 complementary to the key locking elements 104 on the base unit 100. The base unit 100, the mouthpiece 200 and the aerosol head 300 are interconnectable.

The assembly of the base unit 100 with mouthpiece 200 and aerosol head 300 is achieved by insertion of the first segment 200a of the mouthpiece 200 into the groove 103 of the base unit 100, followed by placement of the aerosol head 300 over the first segment 200a of the mouthpiece 200 and engagement of the key locking element(s) 303 of the aerosol head 300 with the complementary element(s) 104 of the base unit 100 through gentle pressure both on the aerosol head and on the base unit. These few assembly steps are intended to produce air-tight connections between the air outlet opening 102 of the base unit 100 and the air intake opening 201 of the first segment 200a of the mouthpiece and also between the aerosol generator 301 and the lateral opening 202 in the first segment 200a of the mouthpiece 200. Furthermore, the aerosol generator 301 is positioned in the aerosol head 300 such that the aerosol generator 301, upon engagement of the key locking element(s) with the complementary element(s), is at least partially inserted into the lateral opening 202 of the first segment 200a of the mouthpiece 200.

Through the outlet opening 102 of the base unit 100, air flows into the air channel 207 of the mouthpiece 200 via the air intake opening 201 of the first segment 200a of the mouthpiece 200. The air outlet opening 102 is circular and can, for example, be positioned in a central region of the base unit (as shown in Fig. 5 for example). The air outlet opening 102 can comprise a sealing element, for example in the form of a gasket, a sealing lip or bellows, which serves to establish an air-tight connection with the air intake opening 201 of the mouthpiece 200.

The air intake opening 201 comprises a perforated plate or filter plate 29 having a central hole 28, which accommodates the shaft 24 of a modified umbrella valve 20. At the same time, the umbrella 26 is situated in the air channel 207 of the mouthpiece 200 and the stop element 23 is situated in the region of the air outlet opening 102 of the base unit 100 (see also Fig. 3a-3c/Fig. 4a-c). The modified umbrella valve 20 prevents especially airflow formed upon exhalation into the mouthpiece opening from passing through possibly incomplete seals, across the air intake opening 201 and the air outlet opening 102, into the base unit 100.

The base unit 100 can comprise one or more electrical connectors for electrical connection thereof, in the assembled state, with complementary connectors of the aerosol head 300. This is particularly important if the aerosol head 300 comprises an ultrasonic or piezoelectric aerosol generator 301, such as an aerosol generator 301 comprising a vibrating membrane 309 (membrane nebulizer).

The power supplied to the aerosol generator can, for example, be provided by an internal battery or a rechargeable battery housed in the base unit 100 or by an external energy source connected via a cable to the base unit 100.

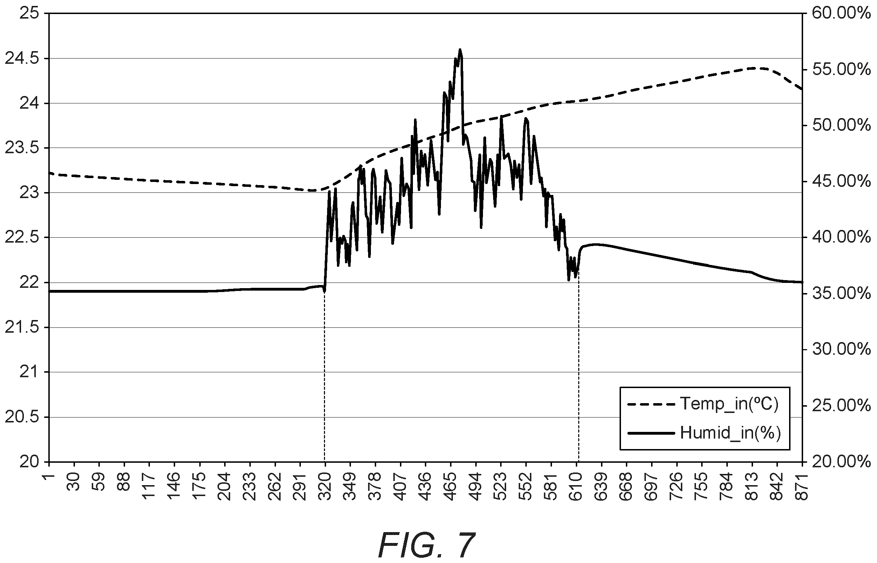

Fig. 7 shows measurement results relating to moisture in the inhaler from the prior art without the modified umbrella valve 20. A moisture sensor was situated in the base part 100 near the pressure sensor 119. The user inhaled through the mouthpiece 200 and exhaled immediately afterwards back into the mouthpiece. From the graph in Fig. 7, it can be seen that the moisture sensor measured an elevated relative air humidity in the exhalation phase (time unit 320 to 610 on the x-axis). From 35% rel. air humidity at 23°C, rel. air humidity rose in the peak up to 57%. On average, the increase in relative air humidity at the sensor during the period of exhalation was about 10%.

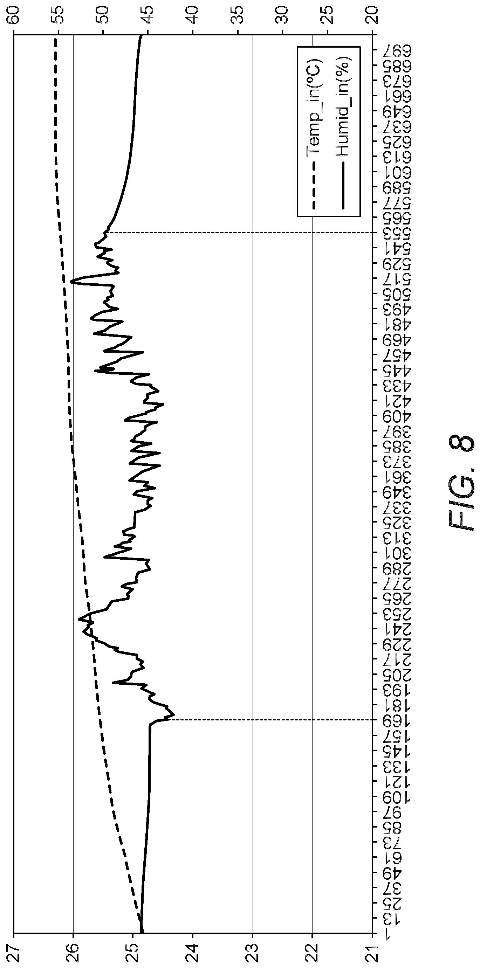

Fig. 8 shows measurement results relating to moisture in the inhaler from the prior art with a modified umbrella valve 20. Exactly as in the test without a modified umbrella valve, the user inhaled through the mouthpiece and exhaled immediately afterwards. From the graph in Fig. 8, it can be seen that the moisture sensor measured a slightly elevated relative air humidity in the exhalation phase (time unit 169 to 553 on the x-axis). The increase in relative air humidity was distinctly lower than without the modified umbrella valve 20. From 45% rel. air humidity at 25°C, rel. air humidity rose in the peak up to 54%. On average, the increase in relative air humidity at the sensor during the period of exhalation was about 1.4%.

## Claims

1. A breath-controlled inhaler (400) comprising
- an air inlet opening (101),
- a mouthpiece (200) having a mouthpiece opening (203),
- an air channel (110, 207) between air inlet (101) and mouthpiece opening (203), wherein the air channel (110, 207) comprises a mixing chamber (208),
- a reservoir (302) for a liquid or for solid particles,
- an aerosol generator (301) which is connected to the reservoir (302) and converts the liquid or the solid particles from the reservoir (302) into an aerosol and introduces it into the mixing chamber (208),
wherein the breath-controlled inhaler (400) further comprises a modified umbrella valve (20) between the air inlet (101) and mixing chamber (208), wherein the modified umbrella valve (20) comprises a solid shaft (24) having a first end and a second end, wherein at the first end of the shaft (24) is situated a flexible umbrella (26), wherein the second end of the shaft (24) is situated a stop element (23) having an enlarged diameter compared to the diameter of the shaft (24),
**characterized in that** the length of the shaft (24) lies in the range from 20% to 50% of the diameter of the umbrella (26).

2. The breath-controlled inhaler (400) as claimed in claim 1, **characterized in that** the length of the shaft (24) lies in the range from 30% to 40% of the diameter of the umbrella (26).

3. The breath-controlled inhaler (400) as claimed in claim 1, **characterized in that** the diameter of the umbrella (26) lies in the range from 9.5 mm to 10.5 mm and the shaft is at least 2 mm, preferably at least 2.5 mm, particularly preferably at least 3 mm long.

4. The breath-controlled inhaler (400) as claimed in claim 1, **characterized in that** the diameter of the umbrella (26) lies in the range from 9.5 mm to 10.5 mm and the shaft is in the range from 3 mm to 4 mm long.

5. The breath-controlled inhaler (400) as claimed in any of claims 1 to 4, **characterized in that** the umbrella (26) is composed of silicone.

6. The breath-controlled inhaler (400) as claimed in any of claims 1 to 5, **characterized in that** the shaft (24) consists of a metal or of a hard plastic, preferably PEEK (polyetheretherketone) or PC (polycarbonate).

7. The breath-controlled inhaler (400) as claimed in any of claims 1 to 6, **characterized in that** the umbrella (26) is formed integrally with the shaft.

8. The breath-controlled inhaler (400) as claimed in any of claims 1 to 7, **characterized in that** in the air channel (110, 207) is situated a perforated plate (29), and the modified umbrella valve (20) is mounted in a central hole (28) of the perforated plate (29) with the stop element (23) on the upstream side of the perforated plate.

9. The breath-controlled inhaler (400) as claimed in claim 8, **characterized in that** the perforated plate (29) is a filter plate comprising a mesh.

10. The breath-controlled inhaler (400) as claimed in any of claims 1 to 9, **characterized in that** the umbrella (26) of the modified umbrella valve (20) points to the mixing chamber (208) and the stop element (23) points to the air inlet (101).

11. The breath-controlled inhaler (400) as claimed in any of claims 1 to 10, **characterized in that** an air inlet valve (118) is situated between the modified umbrella valve (20) and the air inlet opening (101).

12. The breath-controlled inhaler (400) as claimed in claim 11, **characterized in that** a pressure sensor (119) is situated between the air inlet valve (118) and the modified umbrella valve (20).

13. The breath-controlled inhaler (400) as claimed in any of claims 1 to 12, **characterized in that** the aerosol generator (301) comprises a vibrating membrane (309).

14. The breath-controlled inhaler (400) as claimed in any of claims 1 to 13, **characterized in that** it is composed of a head part (300), a base part (100) and a mouthpiece (200), wherein the head part (300) contains the reservoir (302) and the aerosol generator (301), wherein the base part (100) contains the air inlet opening (101) and a first section of the air channel (110) and also, at the end opposite to the air inlet opening, an air outlet opening (102), and wherein the mouthpiece (200) comprises, between an air intake opening (201) and the mouthpiece opening (203), a second section of the air channel (207) comprising the mixing chamber (208), and wherein the modified umbrella valve (20) is mounted in the air intake opening (201).

15. The use of a modified umbrella valve (20) in an air channel (110, 207) of a breath-controlled inhaler, wherein the modified umbrella valve comprises a solid shaft (24) having a first end and a second end, wherein a flexible umbrella (26) is situated at the first end of the shaft (24), and a stop element (23) is situated at the second end of the shaft (24), wherein the stop element has an enlarged diameter compared to the diameter of the shaft (24), wherein the umbrella valve is situated between an air inlet opening (101) and a mixing chamber (208) for mixing air from the air inlet opening (101) and the aerosol from an aerosol generator (301) connected to the air channel (110, 207), **characterized in that** the lenght of the shaft (24) lies in the range from 20% to 50% of the diameter of the umbrella (26).

## Patentansprüche

1. Atemgesteuerter Inhalator (400) umfassend
- eine Lufteinlassöffnung (101),
- ein Mundstück (200) mit einer Mundstücköffnung (203),
- einen Luftkanal (110, 207) zwischen Lufteinlass (101) und Mundstücköffnung (203), wobei der Luftkanal (110, 207) eine Mischkammer (208) umfasst,
- ein Reservoir (302) für eine Flüssigkeit oder für feste Partikel,
- einen Aerosolgenerator (301), der mit dem Reservoir (302) verbunden ist und die Flüssigkeit oder die Feststoffpartikel aus dem Reservoir (302) in ein Aerosol umwandelt und es in die Mischkammer (208) einführt;
wobei der atemgesteuerte Inhalator (400) ferner ein modifiziertes Schirmventil (20) zwischen dem Lufteinlass (101) und der Mischkammer (208) umfasst, wobei das modifizierte Schirmventil (20) einen massiven Schaft (24) mit einem ersten Ende und einem zweiten Ende umfasst, wobei sich an dem ersten Ende des Schafts (24) ein flexibler Schirm (26) befindet, wobei sich das zweite Ende des Schafts (24) in einem Anschlagelement (23) befindet, das einen vergrößerten Durchmesser im Vergleich zu dem Durchmesser des Schafts (24) aufweist,
**dadurch gekennzeichnet, dass** die Länge des Schafts (24) in dem Bereich von 20 % bis 50 % des Durchmessers des Schirms (26) liegt.

2. Atemgesteuerter Inhalator (400) nach Anspruch 1, **dadurch gekennzeichnet, dass** die Länge des Schafts (24) in dem Bereich von 30 % bis 40 % des Durchmessers des Schirms (26) liegt.

3. Atemgesteuerter Inhalator (400) nach Anspruch 1, **dadurch gekennzeichnet, dass** der Durchmesser des Schirms (26) in dem Bereich von 9,5 mm bis 10,5 mm liegt und der Schaft wenigstens 2 mm, vorzugsweise wenigstens 2,5 mm, besonders bevorzugt wenigstens 3 mm lang ist.

4. Atemgesteuerter Inhalator (400) nach Anspruch 1, **dadurch gekennzeichnet, dass** der Durchmesser des Schirms (26) in dem Bereich von 9,5 mm bis 10,5 mm liegt und der Schaft in dem Bereich von 3 mm bis 4 mm lang ist.

5. Atemgesteuerter Inhalator (400) nach einem der Ansprüche 1 bis 4, **dadurch gekennzeichnet, dass** der Schirm (26) aus Silikon besteht.

6. Atemgesteuerter Inhalator (400) nach einem der Ansprüche 1 bis 5, **dadurch gekennzeichnet, dass** der Schaft (24) aus einem Metall oder aus einem harten Kunststoff, vorzugsweise PEEK (Polyetheretherketon) oder PC (Polycarbonat), besteht.

7. Atemgesteuerter Inhalator (400) nach einem der Ansprüche 1 bis 6, **dadurch gekennzeichnet, dass** der Schirm (26) einstückig mit dem Schaft gebildet ist.

8. Atemgesteuerter Inhalator (400) nach einem der Ansprüche 1 bis 7, **dadurch gekennzeichnet, dass** in dem Luftkanal (110, 207) eine Lochplatte (29) angeordnet ist und das modifizierte Schirmventil (20) in einem zentralen Loch (28) der Lochplatte (29) mit dem Anschlagelement (23) an der stromaufwärtigen Seite der Lochplatte angebracht ist.

9. Atemgesteuerter Inhalator (400) nach Anspruch 8, **dadurch gekennzeichnet, dass** die Lochplatte (29) eine Filterplatte umfassend ein Netz ist.

10. Atemgesteuerter Inhalator (400) nach einem der Ansprüche 1 bis 9, **dadurch gekennzeichnet, dass** der Schirm (26) des modifizierten Schirmventils (20) zu der Mischkammer (208) zeigt und das Anschlagelement (23) zu dem Lufteinlass (101) zeigt.

11. Atemgesteuerter Inhalator (400) nach einem der Ansprüche 1 bis 10, **dadurch gekennzeichnet, dass** zwischen dem modifizierten Schirmventil (20) und der Lufteinlassöffnung (101) ein Lufteinlassventil (118) angeordnet ist.

12. Atemgesteuerter Inhalator (400) nach Anspruch 11, **dadurch gekennzeichnet, dass** zwischen dem Lufteinlassventil (118) und dem modifizierten Schirmventil (20) ein Drucksensor (119) angeordnet ist.

13. Atemgesteuerter Inhalator (400) nach einem der Ansprüche 1 bis 12, **dadurch gekennzeichnet, dass** der Aerosolgenerator (301) eine vibrierende Membran (309) umfasst.

14. Atemgesteuerter Inhalator (400) nach einem der Ansprüche 1 bis 13, **dadurch gekennzeichnet, dass** er aus einem Kopfteil (300), einem Bodenteil (100) und einem Mundstück (200) zusammengesetzt ist, wobei das Kopfteil (300) das Reservoir (302) und den Aerosolgenerator (301) enthält, wobei das Bodenteil (100) die Lufteinlassöffnung (101) und einen ersten Abschnitt des Luftkanals (110) sowie an dem Ende gegenüber der Lufteinlassöffnung eine Luftauslassöffnung (102) enthält, und wobei das Mundstück (200) zwischen einer Lufteinlassöffnung (201) und der Mundstücköffnung (203) einen zweiten Abschnitt des Luftkanals (207) umfasst, der die Mischkammer (208) umfasst, und wobei das modifizierte Schirmventil (20) in der Lufteinlassöffnung (201) angebracht ist.

15. Verwendung eines modifizierten Schirmventils (20) in einem Luftkanal (110, 207) eines atemgesteuerten Inhalators, wobei das modifizierte Schirmventil einen massiven Schaft (24) mit einem ersten Ende und einem zweiten Ende umfasst, wobei ein flexibler Schirm (26) an dem ersten Ende des Schafts (24) angeordnet ist und ein Anschlagelement (23) an dem zweiten Ende des Schafts (24) angeordnet ist, wobei das Anschlagelement einen gegenüber dem Durchmesser des Schafts (24) vergrößerten Durchmesser aufweist,
wobei das Schirmventil zwischen einer Lufteinlassöffnung (101) und einer Mischkammer (208) zum Mischen von Luft aus der Lufteinlassöffnung (101) und dem Aerosol aus einem mit dem Luftkanal (110, 207) verbundenen Aerosolgenerator (301) angeordnet ist, **dadurch gekennzeichnet, dass** die Länge des Schafts (24) in dem Bereich von 20 % bis 50 % des Durchmessers des Schirms (26) liegt.

## Revendications

1. Inhalateur commandé par la respiration (400) comprenant
- une ouverture d'entrée d'air (101),
- un embout buccal (200) comportant une ouverture d'embout buccal (203),
- un canal d'air (110, 207) entre l'entrée d'air (101) et l'ouverture d'embout buccal (203), le canal d'air (110, 207) comprenant une chambre de mélange (208),
- un réservoir (302) pour un liquide ou des particules solides,
- un générateur d'aérosol (301) qui est relié au réservoir (302) et convertit le liquide ou les particules solides provenant du réservoir (302) en aérosol et les introduit dans la chambre de mélange (208),
l'inhalateur commandé par la respiration (400) comprenant en outre un clapet parapluie modifié (20) entre l'entrée d'air (101) et la chambre de mélange (208), le clapet parapluie modifié (20) comprenant un arbre plein (24) présentant une première extrémité et une seconde extrémité, au niveau de la première extrémité de l'arbre (24) se trouvant un parapluie flexible (26), au niveau de la seconde extrémité de l'arbre (24) se trouvant un élément de butée (23) présentant un diamètre élargi par rapport au diamètre de l'arbre (24),
**caractérisé en ce que** la longueur de l'arbre (24) est dans la plage comprise entre 20 % et 50 % du diamètre du parapluie (26).

2. Inhalateur commandé par la respiration (400) selon la revendication 1, **caractérisé en ce que** la longueur de l'arbre (24) est dans la plage comprise entre 30 % et 40 % du diamètre du parapluie (26).

3. Inhalateur commandé par la respiration (400) selon la revendication 1, **caractérisé en ce que** le diamètre du parapluie (26) est dans la plage comprise entre 9,5 mm et 10,5 mm et l'arbre a une longueur d'au moins 2 mm, de préférence d'au moins 2,5 mm, de manière particulièrement préférée d'au moins 3 mm.

4. Inhalateur commandé par la respiration (400) selon la revendication 1, **caractérisé en ce que** le diamètre du parapluie (26) est dans la plage comprise entre 9,5 mm et 10,5 mm et **en ce que** l'arbre a une longueur dans la plage comprise entre 3 mm et 4 mm.

5. Inhalateur commandé par la respiration (400) selon l'une quelconque des revendications 1 à 4, **caractérisé en ce que** le parapluie (26) est composé de silicone.

6. Inhalateur commandé par la respiration (400) selon l'une quelconque des revendications 1 à 5, **caractérisé en ce que** l'arbre (24) est constitué d'un métal ou d'une matière plastique dure, de préférence de PEEK (polyétheréthercétone) ou de PC (polycarbonate).

7. Inhalateur commandé par la respiration (400) selon l'une quelconque des revendications 1 à 6, **caractérisé en ce que** le parapluie (26) est formé d'un seul tenant avec l'arbre.

8. Inhalateur commandé par la respiration (400) selon l'une quelconque des revendications 1 à 7, **caractérisé en ce qu'**une plaque perforée (29) est située dans le canal d'air (110, 207), et le clapet parapluie modifié (20) est monté dans un trou central (28) de la plaque perforée (29) avec l'élément d'arrêt (23) sur le côté amont de la plaque perforée.

9. Inhalateur commandé par la respiration (400) selon la revendication 8, **caractérisé en ce que** la plaque perforée (29) est une plaque filtrante comprenant un treillis.

10. Inhalateur commandé par la respiration (400) selon l'une quelconque des revendications 1 à 9, **caractérisé en ce que** le parapluie (26) du clapet parapluie modifié (20) est dirigé vers la chambre de mélange (208) et l'élément d'arrêt (23) est dirigé vers l'entrée d'air (101).

11. Inhalateur commandé par la respiration (400) selon l'une quelconque des revendications 1 à 10, **caractérisé en ce qu'**un clapet d'entrée d'air (118) est situé entre le clapet parapluie modifié (20) et l'ouverture d'entrée d'air (101).

12. Inhalateur commandé par la respiration (400) selon la revendication 11, **caractérisé en ce qu'**un capteur de pression (119) est situé entre le clapet d'entrée d'air (118) et le clapet parapluie modifié (20).

13. Inhalateur commandé par la respiration (400) selon l'une quelconque des revendications 1 à 12, **caractérisé en ce que** le générateur d'aérosol (301) comprend une membrane vibrante (309).

14. Inhalateur commandé par la respiration (400) selon l'une quelconque des revendications 1 à 13, **caractérisé en ce qu'**il est composé d'une partie tête (300), d'une partie base (100) et d'un embout buccal (200), la partie tête (300) contenant le réservoir (302) et le générateur d'aérosol (301), la partie base (100) contenant l'ouverture d'entrée d'air (101) et une première section du canal d'air (110) et également, au niveau de l'extrémité opposée à l'ouverture d'entrée d'air, une ouverture de sortie d'air (102), et l'embout buccal (200) comprenant, entre une ouverture d'admission d'air (201) et l'ouverture d'embout buccal (203), une seconde section du canal d'air (207) comprenant la chambre de mélange (208), et le clapet parapluie modifié (20) étant monté dans l'ouverture d'admission d'air (201).

15. Utilisation d'un clapet parapluie modifié (20) dans un canal d'air (110, 207) d'un inhalateur commandé par la respiration, le clapet parapluie modifié comprenant un arbre plein (24) ayant une première extrémité et une seconde extrémité, un parapluie flexible (26) étant situé au niveau de la première extrémité de l'arbre (24), et un élément de butée (23) étant situé au niveau de la seconde extrémité de l'arbre (24), l'élément de butée présentant un diamètre élargi par rapport au diamètre de l'arbre (24),
le clapet parapluie étant situé entre une ouverture d'entrée d'air (101) et une chambre de mélange (208) pour mélanger l'air provenant de l'ouverture d'entrée d'air (101) et l'aérosol provenant d'un générateur d'aérosol (301) relié au canal d'air (110, 207), **caractérisé en ce que** la longueur de l'arbre (24) est dans la plage comprise entre 20 % et 50 % du diamètre du parapluie (26).
